# EUROPEAN PATENT APPLICATION

(11) **EP 0 618 294 A1**
(43) Date of publication of application: **05.10.1994**
(21) Application number: 94104006.5
(22) Date of filing: 15.03.1994
(51) Int. Cl.: C12N 15/31, C12N 1/21, C12P 21/02, C07K 13/00

(54) **Expression of pertussis holotoxin in bordetella pertussis**

(30) Priority: 15.03.1993 US 31619
(71) Applicant: AMERICAN CYANAMID COMPANY, Wayne New Jersey 07470 (US)
(72) Inventor: Baker, Steven M., Rochester, New York 14623 (US); Deich, Robert A., Rochester, New York 14623 (US)
(74) Representative: Wächtershäuser, Günter, Prof. Dr.

(57) **Abstract**

The invention relates to a cloned region of the *Bordetella pertussis* genome located 3' of the *ptx* operon encoding factors required for expression, assembly or secretion of pertussis holotoxin. Methods for obtaining increased levels of holotoxin production using homologous and heterologous hosts are also described.

## Description

### Background of the Invention

*Bordetella pertussis* is the primary causative agent of pertussis, or whooping cough, an acute infection of the respiratory tract. Pertussis occurs worldwide and is most severe when it infects unimmunized infants. Currently available vaccines (whole cell and partially purified acellular) are believed to have approximately 80-90% efficacy in the first few years after immunization. Effective immunization declines, in the case of whole cell vaccines, to almost no efficacy by 12 years post-immunization. The duration of protection provided by the acellular vaccine is unknown. The currently available vaccines are accompanied by a number of adverse reactions, some of which are severe or life-threatening. These severe reactions can include high fever, seizures, a shock-like hyporesponsive state, encephalopathy and severe allergic reactions. In addition, individuals completely immunized with these vaccines can still develop pertussis. A purified component vaccine specific to the pertussis holotoxin would be useful for developing specific immunity to *B. pertussis* while minimizing potential adverse side effects caused by the currently available complex whole-cell or partially purified acellular vaccines.

One of the limitations of a purified component *B. Pertussis* vaccine is the time and expense involved in the growth and processing of large fermentor volumes of *B. pertussis* required to obtain sufficient amounts of pertussis holotoxin (PT) or mutated forms of the toxin protein, known as cross reactive materials or CRMs. Several investigators have attempted to overcome this limitation by overexpression of PT using either homologous expression systems in *B. pertussis*, or in closely related *B. parapertussis* or *B. bronchiseptica* species (Lee, C.K. et al., Infect. Immun. 57:1413-1418 (1989)), or by utilizing heterologous expression systems such as *E. coli* or *B. subtilis* (Burnette, W.N. et al., Bio/Technology 6:699-705 (1988); Locht, C. and J.M. Keith, Science 232:1258-1264 (1986); Nicosta, A., et al., Proc. Natl. Acad. Sci. USA 83:4631 (1986)). Unfortunately, these efforts have failed to provide any system capable of consistently yielding amounts of PT holotoxin significantly greater than the amount obtained from cultures of wild type *B. pertussis*.

### Summary of the Invention

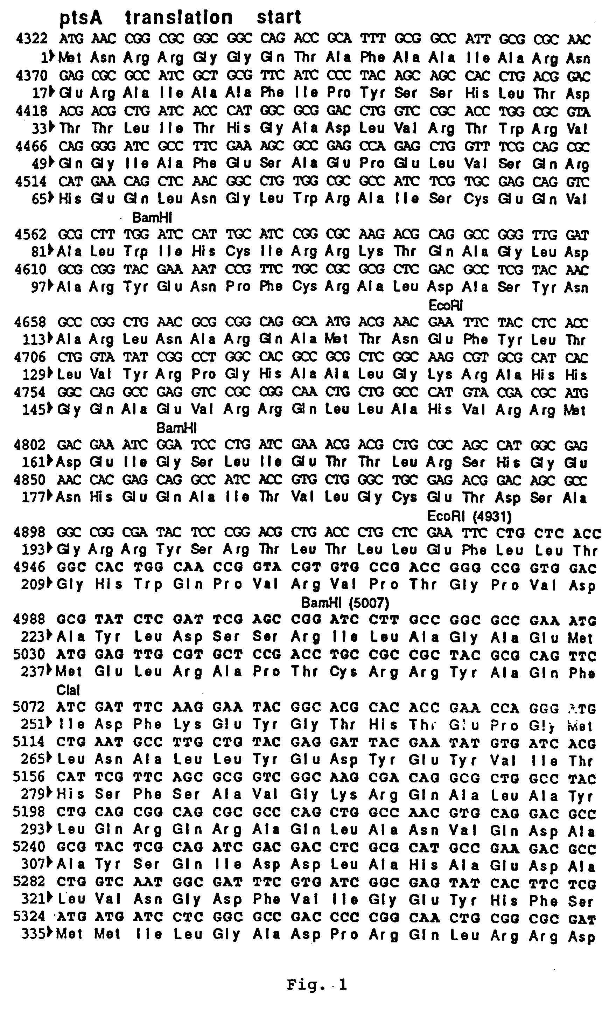

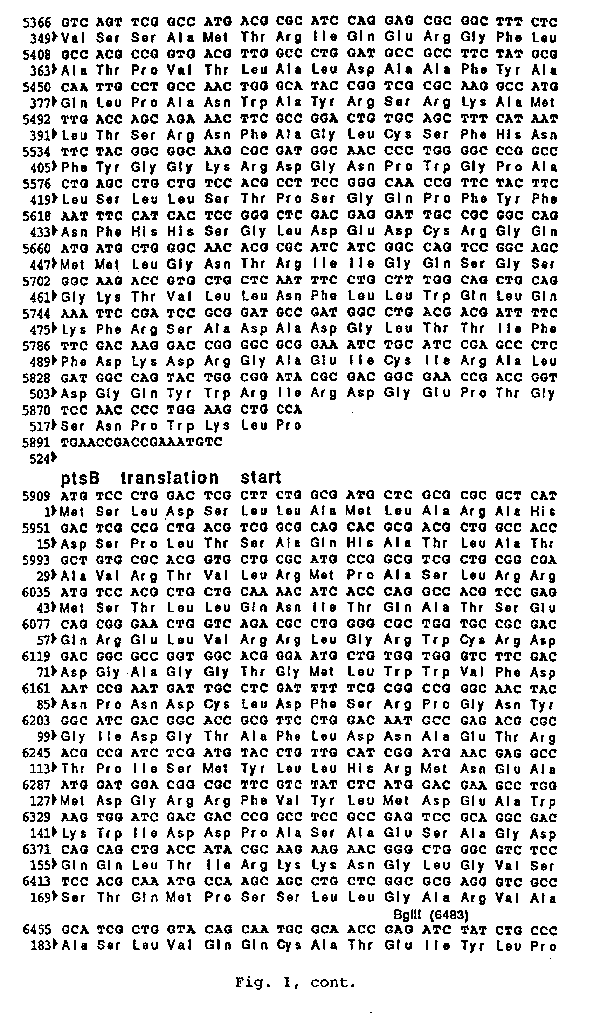

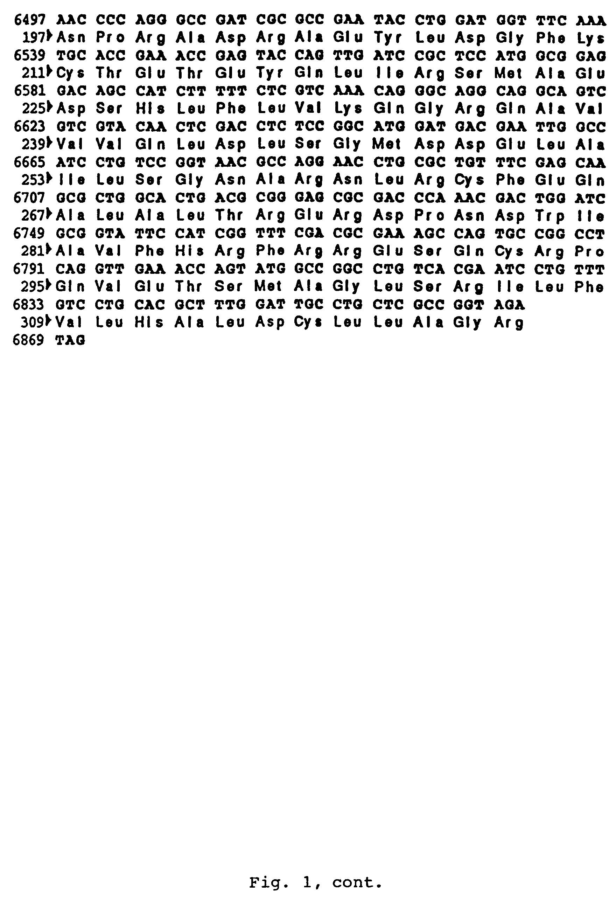

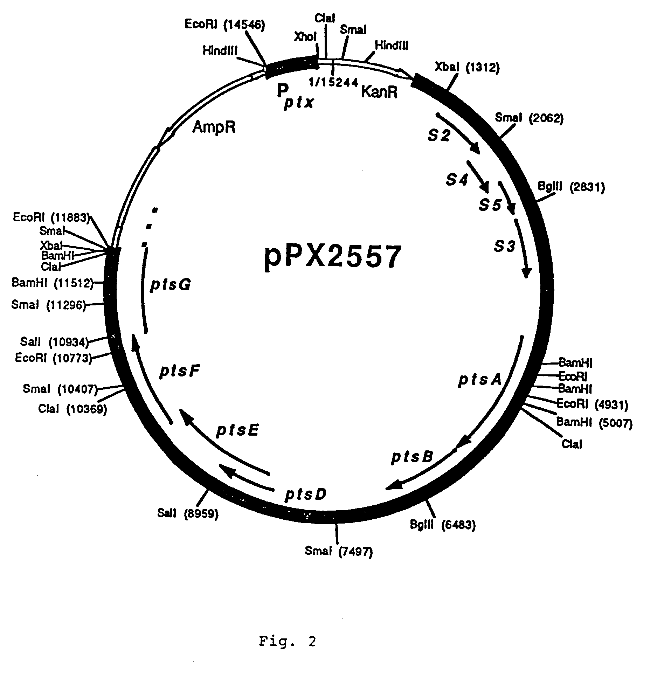

The present invention is based upon the identification of a cloned region of the *B. pertussis* genome. This includes a purified or partially purified nucleic acid sequence comprising an approximately 8kb region of the *B. pertussis* genome, defined herein as the pts region, located immediately 3' (downstream) of the *B. pertussis ptx* operon. This cloned region encodes factors required for expression, assembly or secretion of pertussis holotoxin. The nucleic acid sequence comprises at least two genes, *pts*A and *pts*B, consisting essentially of the nucleotide sequence as shown in Fig. 1 and SEQ ID NO: 1. At least four additional open reading frames have been identified and are included in the cloned *B. pertussis* DNA sequence from a region of the genome 3' of *pts*B, represented in the restriction map shown in Fig. 2. Nucleic acid sequences complementary to all or a portion of the sequence described by SEQ ID NO: 1 and nucleic acid sequences which hybridize under stringent conditions to all or a portion of the sequence described by SEQ ID NO: 1 or its complement are also embraced by the present invention.

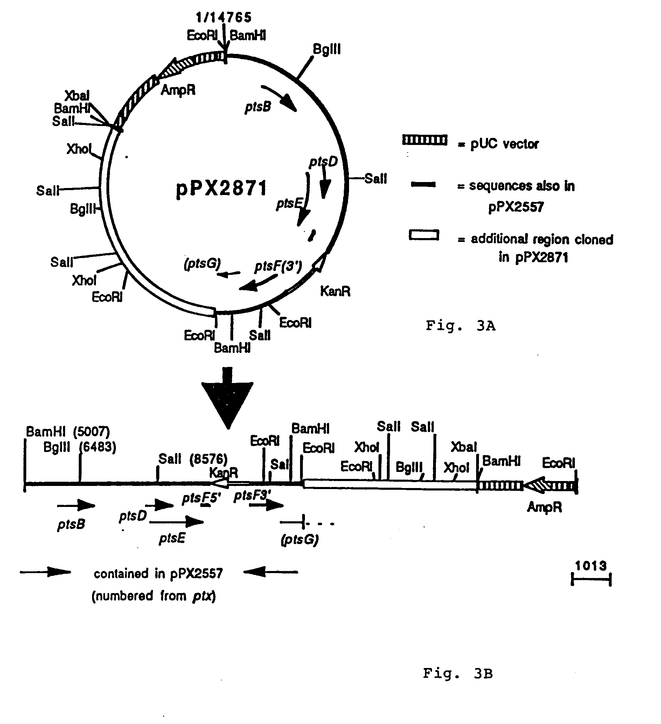

A nucleic acid sequence comprising an approximately 4.5kb region of the *B. pertussis* genome 3' of and contiguous with the approximately 8kb region 3' of *ptx* has been isolated and identified. This region, represented by the restriction map shown in Figs. 2A and 3B, is also described.

In addition to the cloned *B. pertussis* sequences described herein, the present invention includes plasmid constructs comprising the cloned *B. pertussis* sequences, as well as hosts harboring these plasmid constructs. These constructs contain both the approximately 8kb region of the *B. pertussis* genome 3' of the *ptx* operon, and the approximately 4.5kb region further 3' and contiguous with the 8kb region.

In another embodiment, the present invention includes methods for achieving expression and secretion of *B. pertussis* holotoxin in a homologous or heterologous host. According to this embodiment, regions of *pts* necessary or useful for expression and/or secretion of holotoxin in a heterologous host are introduced into the heterologous host in combination with the *ptx* region encoding *B. pertussis* holotoxin. The host is then maintained under conditions suitable for expression and secretion of holotoxin. Using this method expression and/or secretion of holotoxin can be regulated (e.g., up or down regulated) by, for example, placing one or more regions of *pts* under the transcriptional control of a heterologous promoter, increasing *pts* and *ptx* gene dosage, or improving the activity of transcriptional activators of *ptx*, such as *Bvg*A. These methods of regulating expression and/or secretion of holotoxin can be used to produce overexpressing strains of *B. pertussis*, or to produce overproducing homologous or heterologous strains.

A further embodiment of the present invention includes methods for producing large quantities of *B.* *pertussis* holotoxin for use in vaccine production. These methods include growth of a *ptx* overexpressing strain of *B. pertussis*, followed by purification of the holotoxin from the medium. Also included is holotoxin production employing a more rapidly growing and/or overexpressing homologous or heterologous host strain containing *ptx* under the control of a heterologous promoter (e.g., *tac*) and regions of *pts* necessary or useful for expression and secretion of holotoxin from the host strain. Growth of the host strain is then followed by isolation of holotoxin from the growth medium.

### Brief Description of the Drawings

Fig. 1 depicts sequence data from a region of the *B. pertussis* genome 3' of the *ptx* region, beginning at the *ptsA* translation start site at base 4322 (where base 1 is the beginning of the cloned *ptx* region); SEQ ID NO: 1.

Fig. 2 is a restriction map of the plasmid pPX2557 containing *B. pertussis* genomic DNA 3' of the *ptx* operon.

Fig. 3A is a restriction map of pPX2871 containing *B. pertussis* chromosomal sequences 3' of the sequences cloned in pPX2557.

Fig. 3B is a linear restriction map of the *B. pertussis* genomic region cloned in pPX2871 (open box) showing the approximate locations of selected restriction sites numbered from the beginning of the cloned *ptx* sequences (the numbering omitting the approximately 1.2kb contributed by the Kan^{r} gene insert).

Fig. 4 is a schematic depiction of th*e B. pertussis pts*A and *pts*B mutants and cloned mutant genomic sequences.

### Detailed Description of the Invention

A portion of the *B. pertussis* genome encoding products essential for transcription of the genes encoding the hexameric pertussis holotoxin (referred to as PT or holotoxin), and extracellular export of assembled holotoxin has been cloned and characterized. The cloned portion of the genome is located downstream (3') of *ptx* (the region encoding holotoxin subunits) and extends from approximately base 4322 (base 1 is defined as the beginning of *ptx* sequences) to approximately base 16471. This region is cloned in two plasmids, pPX2557 and pPX2871. pPX2557 is shown in Fig. 2 and includes previously characterized nucleic acid sequences (Nicosia, A. et al., Proc. Natl. Acad. Sci. USA 83:4631-4635 (1986)) from the beginning of *ptx* extending into the beginning of an open reading frame designated herein as *pts*A. pPX2557 also includes a previously unreported region of the *B. pertussis* genome extending 3' through the rest of *pts*A and through at least 4 more complete open reading frames (designated *pts*B, *pts*D, *pts*E and *pts*F) and includes an incomplete open reading frame (*pts*G) ending at approximately base 11883. In the restriction map of plasmid pPX2557 shown in Fig. 2, the dark filled regions represent the *ptx* operon and the cloned 3' region of the *B. pertussis*. Unfilled regions represent either pUC vector DNA or the kanamycin resistance (Kan^{r}) gene (from pUC4K) used to disrupt the chromosomal *ptx* S1 gene of the strain used as a source of DNA for the cloning. Other symbols depict the pertussis toxin subunits (S2-S5), the *ptx* promoter (Pₚₜₓ), the *pts*A and *pts*B genes, and open reading frames preliminarily designated *pts*D, *pts*E, *pts*F and *pts*G. Previously unpublished sequence data extends from the *Eco*RI site at 4931 to the *E. coil* site at position 11883.

Plasmid pPX2871 is shown in Fig. 3A and includes the last approximately 6876 bp of *B. pertussis* sequence contained in pPX2557, and the next approximately 4.5kb of the *B. pertussis* genome. A restriction map of this plasmid is shown in Fig. 3A. The solid lines in the map represent regions of *pts* included in pPX2557. The hatched regions represent vector plasmid pT3T7 DNA (a pUC-derived vector). The arrow marked "kan^{r}" denotes the site at which the kanamycin resistance marker was integrated into the chromosome of the strain (designated as PBCC554/pPX2579) used as a source of DNA for the cloned sequences. The open boxed region depicts approximately 4.5 kb of the cloned region present in pPX2871. Fig. 3B is a linear restriction map of the *B. pertussi*s sequences cloned in pPX2871. The open boxed region depicts the region of the *B. pertussis* genome not previously cloned in pPX2557. The locations of the restriction sites within this region are approximate. The solid line at the beginning represents the region previously cloned in pPX2557 which encodes an incomplete open reading frame (*pts*G) homologous to a *vir*B11 protein representative of a family of ATP-dependent translocases involved in transport of large proteins across the outer membrane.

At least six different open reading frames (or genes) are herein identified within the cloned sequences (Fig. 2). As described in greater detail in the following examples, this portion of the genome encodes at least two, and probably more genes that are required for synthesis and extracellular export of holotoxin. The entire cloned region downstream of *ptx* beginning at base 4322 is designated *pts*, for pertussis toxin secretion genes. Two genes located in this region have been designated *pts*A and *pts*B (Fig. 1; SEQ ID NO: 1). The start site of translation for *pts*A is located at base 4322, and the open reading frame continues until a stop codon located at base 5891. *pts*A is required for export of the assembled holotoxin from the periplasm, and encodes a predicted amino acid sequence that is homologous to the predicted amino acid sequence of the *vir*B4 gene product of *Agrobacterium tumefaciens* Ti plasmid (Ward, J.E., et al., J. Bact. 263(12):5804-5814 (1988)). The *pts*B translation start site is located at base 5909 and the open reading frame continues until the stop codon located at base 6869. *pts*B, (located immediately 3' to *pts*A) is required for transcription of *ptx* from its cognate promoter. *pts*B encodes a predicted amino acid sequence that is homologous to the predicted amino acid sequence of the *vir*B5 gene product of *A. tumefaciens* Ti plasmid.

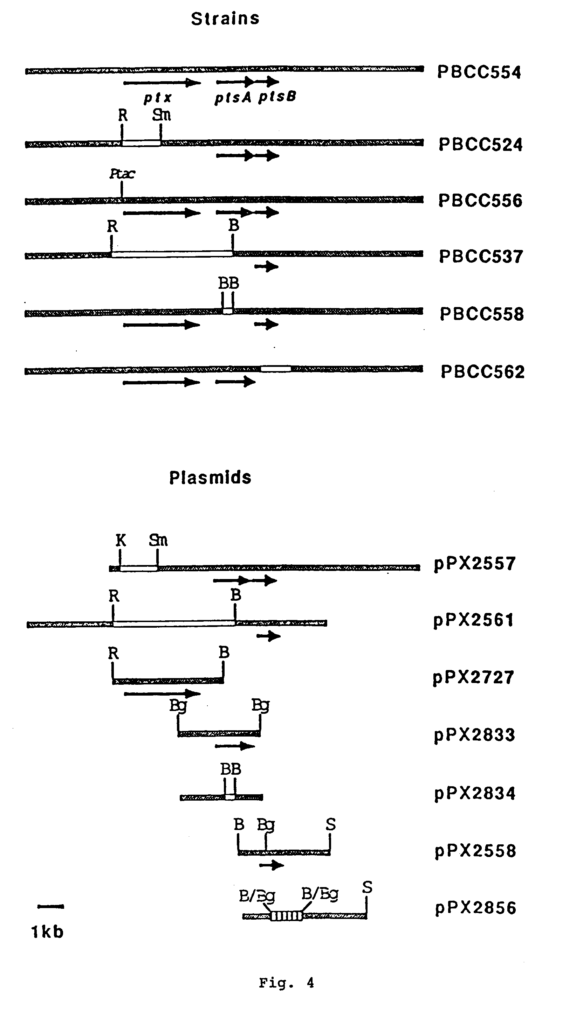

Disruption of the *pts*A gene results in a severe depletion of PT in the culture supernatant of the mutant strain. However, the periplasmic PT of the *pts*A mutant is assembled into a form that is qualitatively similar to the periplasmic PT of *pts*A wild type strains. These results indicate that the *pts*A gene product is primarily involved in the secretion of assembled holotoxin from the periplasm across the outer membrane. Because the steady state level of *ptx* mRNA is not affected by the *pts*A mutation, the approximate 30-fold reduction in the level of PT synthesized by the *pts*A mutant is probably the consequence of post-transcriptional regulation. The *pts*A mutation does not appear to alter expression of other genes, including the *bvg* (*vir*) related functions involving *fha*B (a gene required for expression of the virulence factor, filamentous hemagglutinin (FHA)) or *cya*A (the gene encoding the virulence factor, hemolysis, an adenylate cyclase). The inability of the integrated pPX2833 plasmid containing *pts*A (Fig. 4) to completely restore the secretion defect created by disruption of *pts*A (in PBCC561) can be explained by inactivation of *B. pertussis* genes further downstream of *pts*A, due to vector DNA sequences, or by the presence of a partial protein product (synthesized from the disrupted *pts*A) that interferes with normal *pts*A function. Alternatively, the inability of pPX2833 to complement the *pts*A mutation can also indicate that the *B. pertussis* DNA contained on this plasmid is not integrated in the proper chromosomal context for *cis* regulated expression of *pts*A to occur. The same *pts*A-containing fragment from pPX2833 can be used in plasmid pPX2777 to replace the disrupted chromosomal *pts*A region in the PBCC558 (*pts*A) mutant, without integration of vector DNA, and can completely restore wild type function.

A second gene, *pts*B, encodes a product which is homologous to the predicted *vir*B5 gene product of *A. tumefaciens* and is essential for expression of PT. Insertion of the Kan^{r} marker into *pts*B results in the lack of any detectable transcription of *ptx* and, hence, the lack of detectable PT protein. Disruption of the *pts*B gene does not have any other apparent phenotype. Chromosomal integration of *pts*B, as part of pPX2558, complements the accumulation or expression defect in roughly half of the PBCC562 transformants, indicating that expression of *pts*B is also dependent upon the chromosomal context *pts*B. The absence of any detectable *ptx* mRNA in PBCC562 indicates that the *pts*B gene product is involved in transcription initiation or elongation of the *ptx* mRNA. Although expression of *ptx* (and *cya*A) is known to require at least one factor in addition to BvgA (Goyard, S. and Ullmann, A., FEMS Microbiol. Lett. 61:251-256 (1991); Miller, J.F. et al., J. Bacteriol. 171:6345-6348 (1989)), the factor (or factors) required has not been clearly identified. The *pts*B gene product is not required for expression of *cya*A, making it unlikely that *pts*B encodes Act, the *ptx* and *cya*A promoter binding protein identified by Huh and Weiss (Huh, Y.J. and Weiss, A.A. Infect. Immun. 59:2389-2395 (1991)).

Three additional mutations in the cloned region (identified by gene disruption experiments which localize the mutations to approximately 3 kb, 4 kb and 6kb distal to *ptsA*) have PT export phenotypes similar to that of *ptsA*, indicating that additional gene products encoded by these regions are required for export of holotoxin. DNA sequence analysis of these regions reveals that they contain open reading frames similar to those encoded by the *Agrobacter* Ti plasmid *virB* operon (Ward, J.E., Jr. et al., Proc. Natl. Acad. Sci. USA 88:9350-9354 (1991)). In particular, one of these *B. pertussis* mutations is localized to the extreme 3' end of the *B. pertussis* DNA clone, pPX2557. The DNA sequence of this region reveals an incomplete open reading frame containing homology to th*e virB11* and *pulE* family of putative ATP dependent "secretion kinases," or translocases (Possot, O. et al., Mol. Microbiol. 5:95-101 (1992)). This open reading frame extends into a region of the chromosome now cloned and contained in pPX2871. If the similar genetic organization of the *pts* region and the *Agrobacter vir* region persists, then the sequences cloned in pPX2871 encode an open reading frame containing homology to the transcriptional regulatory *virG* proteins.

The hexameric pertussis toxin protein (PT) is a highly complex bacterial toxin of the A-B type structure. The enzymatically active A monomer (or S1 subunit) is associated with the B oligomer, which contains the S2, S3, S4, and S5 subunits complexed in a 1:1:2:1 molar ratio (Tamura, A., et al. Biochemistry 21:5516-5522 (1982). The genes for these subunits have been cloned and sequenced (Locht, C. and Keith, C.M., Science 232:1258-1264 (1986); Nicosia, A., et al. Proc. Natl. Acad. Sci. USA 83:4631-4635, (1986)). Each of the subunits is thought to be translated from the polycistronic *ptx* mRNA as a precursor protein containing signal sequences. The individual subunit precursors appear to be separately translocated across the cytoplasmic membrane into the periplasmic space where they are assembled into mature holotoxin. Transposon insertion in *ptx*S3 results in accumulation of the other subunits in the periplasmic space of the mutant strain (Marchitto, S.K., et al., Infect. Immun. 55:1309-1313 (1987); Nicosia, A. et al., Infect. Immun. 55:963-967 (1987)). The inability to detect any individual subunits in the culture medium of the *ptx*S3 insertion mutant indicates that the unassembled subunits are not transported across the outer membrane. Assembly and secretion of the B oligomer does not require an intact S1 subunit. Several different mutations in the S1 subunit (including carboxy terminal deletions which prevent association with the B oligomer) give rise to strains that secrete low levels of the B oligomer into the culture medium (Antoine, R. and Locht, C., Infect. Immun. 58:1518-1526 (1990); Pizza, M., et al., J. Biol. Chem. 265:17759-17763 (1990)).

Expression of *ptx* and other virulence factors is subject to regulation by the *bvg*A and *bvg*S gene products of *B. pertussis* (Roy, C.R. et al., J. Bacteriol. 171:6338-6344 (1989); Stibitz, S. et al., Nature 338:266-269 (1989)). These proteins act similarly to two other component signal transducing pathways (Miller, J.F. et al., Science 243:916-922 (1989)) to activate transcription of *ptx*, *fha*B, *cya*A, and other genes encoding virulence factors in *B. pertussis*. Expression of *ptx* has been shown to require factors in addition to the *bvg* gene products (Miller, J.F. et al., J. Bacteriol. 171:6345-6348 (1989)). However, the processing events and factors required for expression, assembly, and secretion of pertussis holotoxin remain to be elucidated.

The inability to obtain overexpression of PT can be attributed largely to the complexity of the *bvg* dependent regulatory system, which controls transcription of the p*tx* operon (Gross, R. and Rappuoli, R., Proc. Natl. Acad. Sci. USA 85:3913-3917 (1988), as well as to the complexity of the multimeric holotoxin protein itself. The *ptx* operon is not transcribed in *E. coli* even when the BvgA transcriptional activator is present (Miller, J.F. et al., J. Bacteriol. 171:6345-6348 (1989). Although each of the individual PT subunits have been expressed individually in *E. coli* (using transcriptional or transcriptional and translational fusions) and then used for assembly of PT in vitro, the process is not readily amenable to large scale production. In addition, the in vitro assembled protein does not exhibit many of the same properties as the native holotoxin (Burnette, W.N. et al., Bio/Technology 6:699-705 (1988).

Attempts to express PT in the faster growing *B. parapertussis* and *B. bronchiseptica* species have been more successful (Lee, C.K. et al., Infect. Immun. 57:1413-1418 (1986). Both of these strains contain homologs of the *ptx* operon but the operon is not expressed because of several mutations in the promoter region (Arico, B. and R. Rappuoli, J. Bacteriol. 169:2847-2853 (1987). These species are able to express PT when an intact *ptx* operon is present. However, secretion or export of PT appears to be strain dependent and in most studies the majority of the toxin remains localized in the periplasm (Lee, C.K. et al., Infect. Immun. 57:1413-1418 (1989); Walker, M.J. et al., Infect. Immun. 59:4238-4248 (1991)). It is postulated that the inability of *B. parapertussis* and *B. bronchiseptica* to efficiently export holotoxin is due to the presence of extracellular transport systems in these organisms which differ from that of *B. pertussis* (Lee, C.K. et al., Infect. Immun. 57:1413-1418 (1989). For these and other reasons, large scale purifications of PT have depended on homologous *B. pertussis* host strains for expression of *ptx*.

The ability to use faster growing heterologous hosts (such as *E. coli* or *Bacillus subtilis*) or overexpressing homologous or related hosts (such as *B. parapertussis* or *B. bronchiseptica*) would substantially reduce the amount of fermentor time required for PT production. The *pts* genes described herein can be used to enhance both expression and export of PT in recombinant homologous or heterologous hosts containing the *ptx* operon. For example, complementation of *pts* function in *B. bronchiseptica* or *B. parapertussis* can be obtained by supplying the *pts* operon on an integrating plasmid. Expression of *pts* genes can be further enhanced by fusing them with very active promoters (for example, the *E. coli tac* promoter). In addition, putative native *pts* homologs (such as those possibly occurring in *B. parapertussis* and *B. bronchiseptica*) can be deleted and replaced with *B. pertussis pts* genes, or with *pts* operatively linked to an active promoter. Another way to increase holotoxin production is to increase the copy number of the *ptx* genes, in conjunction with *pts* genes, in the recombinant homologous or heterologous strain. This can be done, for example, by providing multiple copies of *ptx* and *pts* on an autonomously replicating broad host range plasmid, for example, pRK290 ((Ditta, G. et al., Proc. Natl. Acad. Sci. USA 77:7347-7351 (1980)).

Recombinant strains of *B. pertussis* containing the *E. coli tac* promoter fused to the *ptx* operon export PT independently of some of the factors which normally regulate PT production (e.g., *Bvg*A dependent functions), indicating that the number of factors required for production of PT in a heterologous host can be very limited, and therefore easily provided. Therefore, by using the cloned DNA herein described, homologous, heterologous or related strains can be generated which are capable of high levels of production of pertussis holotoxin. For example, broad host range plasmids containing *ptx* and *pts* operatively linked to one or more active promoters can be introduced into a related or heterologous host strain. *ptx* and *pts* can be present on one or more than one plasmid, and either or both of these operons can be made to integrate into the host genome using, for example, gene replacement techniques. One example of a construct useful for producing an overexpressing strain is a P_{tac}-*ptx pts*A fusion which contains both the *ptx* and *pts*A operons operatively linked to th*e E. coli tac* promoter. This construct can be supplied as an autonomously replicating plasmid, or it can be used to supplant a homologous region in the host cell's genome (for example, *B. parapertussis* or *B. bronchiseptica*) by gene replacement, (S. Stibitz et al., Gene 50:1765-1774 (1986)). Other approaches to producing holotoxin overexpressing (or oversecreting) strains are further described in Example 10. The ability to generate large quantities of holotoxin would make preparation of holotoxin for vaccine production both feasible and economical.

The invention will be further illustrated by the following nonlimiting examples.

### Example 1 - Strains, Plasmids and Media

A brief description of the strains and plasmids described herein is presented in Table 1 and in Fig. 4. Fig. 4 depicts schematically the *B. pertussis* mutant strains and cloned mutant genomic sequences used in the following examples. The thick solid lines represent *B. pertussis* chromosomal regions or chromosomal sequences cloned in plasmid vectors. The plasmid vector sequences are not shown in this diagram and the *B. pertussis* regions cloned are positioned according to the chromosomal orientation. Open boxes depict deleted regions replaced by the kanamycin resistance (Kan^{r}) marker. The filled box (pPX2856) represents insertion of Kan^{r} without deletion of cloned sequences; thick arrows represent intact open reading frames; Ptac depicts the *E. coli* tac promoter fusion in PBCC556; symbols for restriction sites are as follows R, EcoRI; Sm, SmaI; B, BamHI; K, KpnI; Bg, BglII.

*B. pertussis* strains are grown on solid Bordet-Gengou medium (BG; Difco Laboratories, Detroit, MI) containing 15% defibrinated horse blood (Crane Biologics, Syracuse, NY) or in modified Stainer-Scholte liquid medium containing 0.1% 2,6-O- methyl-β-cyclodextrin (Teijin Limited, Tokyo, Japan) as described previously (Kimura, A. et al., Infect. Immun. 58:7-16 (1990)). Cultures grown under modulating conditions contain 50mM MgSO₄ in both solid and liquid media. When antibiotics are included in either type of medium, they are added to the following concentrations: 50µg/ml ampicillin, 25µg/ml kanamycin, 200µg/ml streptomycin, or 10µg/ml tetracycline. Growth of liquid cultures is followed by measuring the optical density (OD) at 650 nm. Mid to late logarithmic phase cultures (OD=1.0 to 1.5) are used for most assays. *E. coli* strains are grown in LB medium (Sambrook, S. et al., 1989 Cold Spring Harbor Laboratory, Cold Spring Harbor, NY) with ampicillin added to 100 µg/ml when required.

### Plasmid Constructions and Genetic Manipulations

Restriction enzymes, T4 DNA ligase, T3 or T7 RNA polymerase, and other enzymes and nucleotides used for DNA or RNA manipulations are obtained from Boehringer Mannheim (Indianapolis, IN), Bethesda Research Laboratories Inc. (Bethesda, MD), or New England Biolabs (Beverly, MA). DNA sequencing is carried out using an Applied Biosystems (Foster City, CA) model 370A DNA Sequencer. Computer searches of the Genbank International Nucleotide Sequence Databank (Intelligenetics Inc., Mountain View, CA) are done on a Macintosh IIci computer (Apple Computer, Cupertino, CA) using MacVector 3.5 software (International Biotechnologies Inc., New Haven CT). All other DNA manipulations are performed using standard conditions (J. Sambrook et al., 1989 Cold Spring Harbor Laboratory, Cold Spring Harbor, NY).

Plasmid pPX2557 (Table 1 and Figs. 1 and 2) is obtained by selecting kanamycin resistant (Kan^{r}) recombinants in pUC19 from an EcoRV genomic digest of *B. pertussis* strain PBCC502 (similar to PBCC524, see Fig. 4) which contains a Kan^{r} marker replacing the 5' region (KpnI to SmaI) of the *ptx* operon. The resulting pPX2557 plasmid contains 2.7kb of the *ptx* operon and 8kb of the adjacent 3' region. A 3.7kb BglII fragment containing *pts*A is subcloned from pPX2557 into the BamHI site of pUC18 to create pPX2833 (Table 1 and Fig. 4). The *pts*A gene in pPX2833 is disrupted by digestion with BamHI and insertion of the Kan^{r} marker which replaces 438bp of the *pts*A coding sequence in the resulting plasmid, pPX2834 (Table 1 and Fig. 4). Plasmid pPX2558 (Table 1 and Fig. 4) contains the *pts*B coding region, included as part of the 4.5kb BamHI-SalI region from pPX2557 subcloned into pUC18. The *pts*B coding region contained in pPX2558 is interrupted by ligating the Kan^{r} marker into the unique BglII to yield pPX2856 (Table 1 and Fig. 4). Plasmid pPX2777 is derived from pN01523 (Pharmacia-LKB, Piscataway, NJ) after destroying the EcoRI and HindIII sites of pNO1523 and ligating a DNA fragment, containing the pUC18 multiple cloning site and *lac*Z region, into the BamHI site of the modified pN01523. The resulting plasmid (pPX2777, Table 1) permits blue/white screening for recombinants and the dominant, Str^{s}, *rps*L allele facilitates the return of genes to Str^{r} *B. pertussis*, similar to the method described for pRTP1 (S. Stibitz et al., Gene 50:1765-1774 (1986)). Plasmid pPX2857 (Table 1 and Fig. 4) is constructed by ligating the 3.7kb BglII fragment, described above, into the unique BamHI site of pPX2777.

Plasmids are introduced into *B. pertussis* by electroporation using a BTX (San Diego, CA) Transfector 100 electroporator equipped with a 0.5mm electrode and Power Plus module. Cells are prepared for electroporation by harvesting mid-logarithmic phase cultures (OD₆₅₀ = 0.8 to 1.0), washing them in 1/4 the culture volume of 1 mM HEPES, pH7.2, and resuspending in 1/50 volume 1 mM HEPES, pH7.2, containing 10% glycerol, prior to storage at -70°C (Zealey, G. et al., FEMS Microbiol. Lett. 50:123-126 (1988)). During electroporation the amplitude is set to obtain a pulse of 28-30kv/cm. Cells are preincubated in liquid medium for 60 minutes at 37°C and plated onto selective medium. For gene disruption experiments, pUC based vectors (which do not replicate in *B. pertussis*) are used and selection is for the Kan^{r} marker used to replace or interrupt the gene of interest. Double recombination events (between the regions of the gene flanking both sides of the marker and the homologous chromosomal sequences) are scored by determining sensitivity to ampicillin. Plasmids derived from the pPX2777 vector are used to replace chromosomal sequences by streaking the Amp^{r} transformants onto streptomycin plates followed by screening for the appropriate phenotype as described previously (Stibitz, S. et al., Gene 50:1765-1774 (1986)).

### RNase Protection

RNA antisense probes are prepared from clones contained in pT3-T7 (Boehringer Mannheim) by transcription of the linearized templates in the presence of (α-³²P-GTP) (Amersham, Arlington Heights, IL). Total cellular RNA is isolated from mid logarithmic phase cells using a modification of the hot phenol method (von Gabain, A. et al., Proc. Natl. Acad. Sci. USA 80:653-657 (1983)). Fifty µg of RNA is incubated with 10⁵ cpm of the radiolabelled probe in a final volume of 30µl of hybridization buffer at 95°C for 3 minutes prior to hybridization overnight at 58°C. Single stranded RNA is digested with RNase A and RNase T1 (Boehringer Mannheim, Indianapolis, IN) as described elsewhere (Kreig, P.A. and Melton, D.A., Meth. Enzymol. 155:397-415 (1987)). The protected RNA is fractionated on a denaturing 8% polyacrylamide gel along with Mspl digested pBR322 radiolabelled size standards.

### Immunochemical Assays

Colony immunoblots of *B. pertussis* are performed by standard procedures (Sambrook, S. et al., 1989 Cold Spring Harbor Laboratory, Cold Spring Harbor, NY) after growing cells on nitrocellulose filters (BA85, Schleicher & Schuell, Keene, NH) for 2-3 days. The presence of PT is detected using a goat anti-pertussis toxin (PT) antibody fraction and horse radish peroxidase (HRP) conjugated rabbit anti-goat antibody (Zymed, San Francisco, CA). Positive colonies are visualized by incubation with 4-chloro-1-naphthol and hydrogen peroxide. PT is adsorbed from culture supernatants onto fetuin agarose (Sigma Chemical Co., St. Louis, MO) as previously described (Kimura, A. et al., Infect. Immun. 58:7-16 (1990)). The adsorbed PT is analyzed by SDS-polyacrylamide electrophoresis (Laemmli, U.K., Nature 227:680-685 (1970)) using 16% SDS-polyacrylamide gels. For Western analysis, whole cell lysates are resolved on 16% SDS-polyacrylamide gels, electroblotted onto nitrocellulose (BA85, Schleicher & Schuell) and probed using goat anti-PT and HRP rabbit anti-goat antibody as described above. Volumes of samples used for SDS-polyacrylamide electrophoresis and Western analyses are normalized to the culture OD. Hemagglutination assays are conducted as previously described (Sato, Y. et al., Infect. Immun. 41:313-320 (1983)) using a 1% suspension of washed goose red blood cells (Crane Biologics, Syracuse, NY).

The level of toxin in the culture supernatant and in the periplasmic space is determined by an antigen capture ELISA. The integrity of the toxin is assessed by ELISA based assays in which the binding ability of the toxin to either fetuin or the PT receptor in Chinese hamster cells (CHO) is measured. Microtiter plates are coated with either goat polyclonal antibodies to PT for the antigen capture ELISA, fetuin for the fetuin binding ELISA, or CHO cell cytoplasmic membranes for the CHO membrane binding ELISA. The CHO cell membranes are isolated (Brennan, M.J., et al., J. Biol. Chem. 263:4895-4899 (1988)) from CHO-K1 cells or from CHO-15B cells. Because the receptors in the ricin-resistant CHO-15B cells is defective and can no longer bind PT (Locht, C. et al., Infect. Immun. 55:2546-2553 (1987)), the difference in the extent of PT binding to the CHO-K1 membranes and to the CHO-15B membranes is taken to represent the specific binding of PT to the receptor.

Unless otherwise specified, the following steps for ELISA analyses are performed at 37°C for 1 hour. Coated plates are blocked with 2% bovine serum albumin (BSA). The culture supernatant as well as the periplasmic fraction are serially diluted and added to the plates for incubation. The bound toxin is probed with a mouse polyclonal serum to PT followed by alkaline phosphatase conjugated goat F(ab')2 anti-mouse immunoglobulins (Tago Inc., Burlingame, CA). The plates are then developed with 1 mg/ml p-nitrophenyl phosphate for 30 minutes at room temperature and the OD at 410 nm is measured.

In each ELISA, purified PT is used to establish a standard curve. The specific binding activity of PT, defined as OD/µg protein, is derived from the linear portion of a plot of net OD versus PT concentration. Based on these standard curves, the toxin concentration of the test samples is calculated. The detection limit is from 1 to 10 ng/ml for the antigen capture ELISA, from 8 to 80 ng/ml for the fetuin binding ELISA, and from 3 to 30 ng/ml for the CHO receptor binding ELISA.

The periplasmic fractions used in the above-described ELISAs are prepared using polymixin B (Sigma, St. Louis, MO) treatment of concentrated whole cells (Pizza, M. et al., J. Biol. Chem. 265: 17759-17763 (1990)). The level of toxin recovered in these fractions is normalized to the relative yield of the periplasmic material as estimated by the β-lactamase activity present in the periplasmic and cellular fractions of PBCC561 and other Amp^{r} control strains.

### Example 2 - Identification of B. pertussis toxin secretion mutants

First, a *ptx* deletion strain is constructed and designed to avoid possible complications resulting from recombination between plasmid encoded *ptx* sequences and homologous sequences that would otherwise be present on the host chromosome. A 4.6kb EcoRI-BamHI *B. pertussis* chromosomal region carrying the entire *ptx* operon and 1.3kb of 3' flanking sequence (Fig. 4) is replaced with the Kan^{r} marker by homologous recombination with the flanking DNA sequences present on pPX2561 (Fig. 4). Gene replacement is verified by Southern hybridization analysis. All of the isolates remain hemolytic and produce filamentous hemagglutinin (FHA) (measured by hemagglutination) and 69K outer membrane protein (OMP) (or pertactin (Roberts, M. et al., Mol. Microbiol. 5:1393-1404 (1991)), as determined by Western analysis) upon repeated subculturing. One of the isolates (PBCC537, Fig. 4) is used as a host to examine the expression of PT from plasmid copies of the *ptx* operon.

After electroporation of PBCC537 with pRK290 based plasmids (Ditta, G. et al., Proc. Natl. Acad. Sci. USA 77:7347-7351 (1980)) that are capable of replicating in *B.* *pertussis* and contain an intact *ptx* operon (for example pPX2727, Fig. 4), Amp^{r} transformants are screened for toxin production by colony immunoblot. All of the hemolytic colonies tested also produce toxin protein as judged by a positive reaction on colony immunoblots. Positive colonies are further tested for the presence of toxin in the culture supernatant by growing small scale liquid cultures and testing the supernatants for toxin by fetuin binding ELISA. On the basis of these tests, all of the isolates tested are unable to secrete holotoxin (or the B-oligomer) into the culture medium. The inability of the PBCC537 transformed strains to secrete toxin is not due to a defect in the plasmids or plasmid borne *ptx* gene; toxin expressed from the same plasmids is secreted from other *B. pertussis* host strains (PBCC524 or similar hosts) that are deleted only for the 5' region of the *ptx* operon.

### Example 3 - Identification and sequence analysis of ptsA

Computer analysis of the DNA sequence deleted in PBCC537 reveals an incomplete open reading frame (ORF) that starts 697bp downstream from the end of the *ptx*S3 coding sequence and extends beyond the end of the published sequence (Nicosia, A. et al., Proc. Natl. Acad. Sci. USA 83:4631-4635 (1986)). A search of Genbank shows that the predicted product of this ORF is homologous to the predicted *vir*B4 gene product which is thought to be part of a multi-protein complex involved in transport of the Ti plasmid DNA across the cell envelope of *Agrobacter tumefaciens* (Ward, J.E., Jr. et al., Proc. Natl. Acad. Sci. USA 88:9350-9354 (1991)). Additional DNA sequencing of the *ptx* 3' ORF contained in pPX2833 reveals a predicted protein of 61Kd that is homologous (29% identity) to *vir*B4 throughout the entire 3'ORF. This region is referred to as the *pts* (pertussis toxin secretion) locus and this specific ORF is referred to as *pts*A.

### Example 4: - Characterization of ptsA function in holotoxin assembly or secretion

To more clearly demonstrate the requirement of *pts*A in the assembly or secretion of PT, the *pts*A region is subcloned as a 3.7kb BglII DNA fragment (pPX2833, Fig. 4), and then an internal BamHI-BamHI fragment of the putative gene is replaced with a marker for kanamycin resistance. The disrupted *pts*A gene contained on pPX2834 (Table 1 and Fig. 4) is used to replace the intact chromosomal copy of *pts*A by electroporation of PBCC554 (Table 1 and Fig. 4) followed by selection on kanamycin and screening for Amp^{s}, hemolytic colonies. All of the hemolytic, Kan^{r}, Amp^{s} colonies test positive for PT by colony immunoblot. Twelve isolates are further screened for secretion of PT by growing small scale cultures and testing the culture supernatants for hemagglutination activity. All twelve are negative for any hemagglutination activity. One of the isolates is designated PBCC558 and saved for further characterization.

Attempts to complement the toxin assembly or secretion defect created in PBCC558 by integrating an intact copy of *pts*A using pPX2833 yield the following results: 15 of 18 hemolytic, Amp^{r} transformants tested remain positive by colony immunoblot, but only marginal hemagglutination activity (titers of 1:2 to 1:4) can be detected in the culture supernatants. One of these transformants is designated PBCC561 (Table 1). Subcloning the same region into pPX2777 (Table 1) demonstrates that the assembly or secretion defect can be completely restored by the 3.7kb BglII fragment. The resulting plasmid, pPX2857 (Table 1) facilitates replacement of the disrupted chromosomal region of PBCC558 without integration of vector sequences. Two isolates of the appropriate phenotype (Amp^{s}, Kan^{s}, Str^{r} and hemolytic) contain levels of hemagglutination activity in culture supenatants that are identical to the PBCC554 parental strain. One of the isolates is PBCC563 (Table 1).

A plasmid containing the Kan^{r} marker ligated into the BglII site located past (3') the predicted end of the *pts*A coding region is constructed in order to test the hypothesis that the incomplete complementation exhibited by PBCC561 may be due to polar effects or interference of the integrated pPX2833 vector sequences on additional genes located downstream of *pts*. The resulting plasmid (pPX2856, Table 1 and Fig. 4) is electroporated into PBCC554. Surprisingly, all of the hemolytic, Kan^{r}, Amp^{s} recombinants are negative for PT expression when screened by colony immunoblots. One of these isolates, PBCC562, is used as a host for electroporation with an integrating plasmid (pPX2558, Table 1 and Fig. 4) containing an intact region corresponding to the site of the Kan^{r} chromosomal insertion. Approximately half of the resulting Amp^{r} transformants tested are positive for PT accumulation by colony immunoblot, however, none of the positive colonies express hemagglutination activity when grown in liquid culture.

### Example 5: - Identification and sequence analysis of ptsB

DNA sequencing of the regions flanking the site of the BglII insertion in pPX2856 and PBCC562 reveals that insertion of the Kan^{r} marker interrupts a second open reading frame (ORF). This second ORF contains four initiation codons within the first 213 nucleotides, the first of which occurs 123bp before the 3' end of the *pts*A coding sequence. If translation starts at the third initiation codon (located 67bp past the end of *pts*A coding region), a predicted protein of 32.5kd, which is 33% identical to the predicted *vir*B5 gene product of *A. tumefaciens*, would be expressed from this region. This gene is designated *pts*B.

### Example 6: - Characterization of ptsA and ptsB function in expression, assembly and secretion of holotoxin

SDS-PAGE and Western analysis of fetuin bound material recovered from culture supernatants from PBCC558, PBCC561 and PBCC562 allows for characterization of the nature of the defects in these mutants by assaying for the presence of secreted holotoxin. The results of SDS-PAGE show that PBCC558 and PBCC561 are depleted for extra-cellular PT in a form capable of binding fetuin and that no detectable PT is recovered from PBCC562. The goat anti-PT polyclonal antibody, which detects primarily the S1 subunit of PT, is used for Western analysis of the fetuin absorbed fractions from the above strains. The presence of the S1 subunit is readily detected in PBCC554 and PBCC563 and is present at lower levels in the two *pts*A mutant strains PBCC558 and PBCC561. The presence of S1 in the fetuin absorbed fraction from the two mutant strains indicates that the low level of PT secreted by these strains is in the form of holotoxin, as opposed to only the B-oligomer, which also binds to fetuin. These results also show that the PBCC562 *pts*B mutant lacks S1. A faint band migrating slightly below the S1 protein is detected by polyclonal antisera in culture supernatants from PBCC562 is also detected in the fetuin absorbed fraction and whole cell lysates of *ptx* deletion strains. This band is therefore most likely due to a low level of a cross reactive protein. The presence of wild type levels of PT and S1 recovered from PBCC563 also shows that repair of the disrupted *pts*A in PBCC563 restores the capacity to secrete wild type levels of PT holotoxin. Western analyses of whole cell lysates of the different strains show that the S1 subunit is detected in lysates of all strains except the PBCC562 *pts*B mutant.

### Example 7: - Characterization of the ptsA mutant phenotype - ptsA mutant is secretion defective

Assays that permit estimation of the amount of PT protein present relative to the amount of functional PT capable of binding to either fetuin coated microtiter plates or CHO cell membrane coated microtiter plates are used to determine whether the defect present in PBCC558 is due to the failure of the strain to assemble the toxin subunits into a form that can bind fetuin or CHO cell membranes, or if the defect is due to an impaired capacity to secrete the assembled holotoxin. The three different assays provide a range of values for the estimated absolute amount of PT present in culture supenatants (Table 2). The values obtained by the fetuin and CHO membrane ELISAs are 1.3 to 1.4 fold higher, respectively, than those obtained by antigen capture ELISA of PBCC554 culture supernatant. However, it is clear that PBCC554 (which serves as a wild type control) contains only a small fraction (less than 5%) of the total PT protein (extracellular plus periplasmic) in the periplasmic fraction, as detected by the antigen capture ELISA, and that the bulk of the PT is recovered in the culture supernatant. The *pts*A mutant, PBCC558, is severely reduced in the total amount of PT and this reduction can be attributed almost entirely to the lack of extracellular PT (Table 2). The small amount (∼3% of wild type level) of extracellular PT that is detected by antigen capture ELISA of PBCC558 culture supernatants also appears functional in the fetuin and CHO cell membrane binding ELISAs (Table 2). The periplasmic PT from PBCC558 does not appear significantly different than the periplasmic PT from PBCC554; about the same amount of PT is recovered from the periplasmic fraction from both strains and approximately the same fraction (0.4-0.5x) is detected by the fetuin binding and CHO binding assays (Table 2).

These results indicate that the defect conferred by the *pts*A mutation present in PBCC558 is not a consequence of defective assembly of PT, but is due to the inability of the mutant strain to secrete the assembled PT holotoxin or B-oligomer. The ELISA results also indicate that the integrated copy of pPX2833 (which contains an intact *pts*A open reading frame) in PBCC561 does not result in any significant increase in the amount of secreted PT. PBCC562 lacks any detectable PT in either the periplasmic or extracellular fraction (Table 2). Similar assays have shown that PBCC563 is very similar to the original parental strain (PBCC554) with respect to the amount of functional PT present in both the periplasm and culture supernatant.

### Example 8: - Identification of the ptsA transcriptional start site

Initial identification of the transcription start site for the mRNA encoding *pts*A uses an antisense RNA probe homologous to a region 89bp before the predicted translation start site and including 370bp of the complementary coding sequence. Using this probe for RNase protection of the mRNA synthesized by the wild type (Tohama) strain grown under non-modulating conditions reveals that the entire length of the probe is protected from RNase digestion, indicating that the transcriptional start site for *pts*A occurs upstream of the region probed. An RNA antisense probe extending further upstream from the translation start (-90 to -698) of *pts*A is used to hybridize to RNA from the wild type strain grown under modulating (+MgSO₄) and non-modulating (-MgSO₄) conditions. The results of this experiment show that the probe is protected only when used to hybridize to RNA from the wild type strain grown under non-modulating conditions. The estimated size of this band (∼600nt) is the size expected if the entire length of the probe, minus ∼160nt corresponding to vector sequences, is protected from RNase digestion. These results indicate that transcription of *pts*A is regulated by the presence MgS0₄, and that initiation of transcription occurs upstream of the region probed (i.e., 5' of the end of *ptx*S3).

### Example 9: - ptsB mutation blocks transcription of ptx or accumulation of ptx mRNA

RNA from the different mutants is hybridized to an RNA antisense probe overlapping the -167 to +327 region of *ptx* transcription initiation site in order to determine if the *pts*A and *pt*sB mutations alter transcription of *ptx*. Results from this experiment show that the RNA from Tohama protects a region of the probe (corresponding to approximately 327nt) expected if transcription initiates at the published transcription initiation site (Nicosia, A. et al., Proc. Natl. Acad. Sci. USA 83: 4631-4635 (1986)). RNA from PBCC554 protects two regions of the probe corresponding to ∼150nt and ∼120nt. The 150nt band is expected if transcription of *ptx* starts at the same transcription initiation site and extends through the R9K mutation present in PBCC554. The 120nt band is the expected digestion product corresponding to the distance from the W261 mutation to the end of the probe. For each of these mutations, the three base pair mismatch of the RNA duplex formed by hybridization of the wild type sequence of the probe with mRNA corresponding to the mutated regions in PBCC554 (and its derivatives), results in digestion by RNase to yield the observed pattern. RNA from PBCC558, PBCC561 and PBCC563 also protects the same regions of the probe from RNase digestion. However, PBCC562 lacks any detectable *ptx* mRNA, indicating that the *pts*B insertion mutation either blocks transcription of *ptx* or prevents accumulation of *ptx* mRNA.

In the *A. tumefaciens* Ti plasmid, the *vir*B gene cluster contains 11 open reading frames (Ward, J.E., Jr. et al., Proc. Natl. Acad. Sci. USA 88:9350-9354 (1991)). It appears likely that the chromosome of *B. pertussis* contains a subset of similar genes or open reading frames that may be organized in a similar fashion and encode homologous proteins. Although only two genes in *B. pertussis* that affect expression of *ptx* have so far been identified, it is likely that more genes are involved. The observation that integration of pPX2558 can restore expression, but not secretion, of PT in the PBCC562 *pts*B mutant, suggests that at least one additional factor (other than the *pts*A or *pts*B gene products) is involved in secretion of the holotoxin. This theory is further substantiated by a recent report by Johnson et al. (Johnson, F.D., et al., p. 28 of the abstracts of the 92nd General Meeting of the American Society of Microbiology, Characterization of a Mutant of Bordetella Pertussis Toxin in the Culture Supernatant (1992)), describing a transposon insertion which maps 3.2kb 3' to *ptx* (near the end of *pts*B) and has a phenotype similar to the *pts*A mutant. In addition, described herein are data indicating that the most distal region (approximately 8kb 3' of *ptx*) cloned in pPX2557 encodes a predicted ORF homologous to the predicted *vir*B11 gene product (Fig. 1).

The manner in which *pts* is regulated is unclear. The results described herein show that, in wild type *B. pertussis*, *pts*A is subject to virulence modulation and is regulated at the transcriptional level in a manner similar to the other *bvg* regulated genes in *B. pertussis*. However, when transcription of *ptx* is uncoupled from *bvg* regulation by fusion to P_{tac} as in PBCC556 (Table 1 and Fig. 4), expression of *pts* secretion function also becomes independent of *bvg* and holotoxin is secreted even when MgSO₄ is included in the growth medium. The inability to detect the 5' end of the *pts*A mRNA within almost 700bp of the translation initiation codon and the observation that transcription of *pts*A appears to be regulated by modulating growth conditions indicate that expression of *pts*A may be regulated by *ptx*. Experiments demonstrating that deletions of the 5' region of *ptx* (including the promoter) can be complemented by plasmid encoded *ptx* (Locht, C., Infect. Immun. 55:1309-1313 (1987) and that insertion of a promoterless cat gene at the 3' end of *ptx*S3 does not affect PT secretion suggest that *pts* may be regulated *in trans* by PT. However, plasmid integration in the *rec*A⁺ host cells used in the plasmid expression experiments may also result in *cis* complementation of the PT secretion function. Furthermore, the presence of the cat gene at the end of *ptx* does not appear to alter transcription of the region downstream of *ptx*. Although it has not yet been determined whether expression of *pts*A is regulated *in cis* by transcription or translation of *ptx*, it is unlikely that *pts*B is regulated by transcription from the *ptx* promoter because the *pts*B gene product itself is required for transcription of *ptx*.

### Example 10: - pts gene products can facilitate overexpression of PT in either homologous or heterologous expression systems

Characterization of the *pts*A mutant indicates that enhanced secretion of PT from the periplasm provides a net increase in the recovery of PT in culture supernatants. Thus, increasing the amount or activity of *pts*A and similar gene products can itself result in enhanced recovery of PT, or can result in increased levels of PT when used in conjunction with one or more of the approaches described previously (e.g., increased *ptx* gene dosage, use of alternate transcriptional/translational signals, or improved activity of transcriptional activators such as *Bvg*A or *Pts*B). This approach to increasing PT expression and secretion is supported by a strain (designated PBCC566) containing the *E. coli tac* promoter fused to the translation initiation site of *ptsA*. This strain exhibits levels of PT in the culture supernatant that are increased above wild type levels.

One of the advantages of utilizing the heterologous Bordetella species, *B. parapertussis* or *B. bronchiseptica* for expression of PT is that the faster growth of these species would substantially reduce the amount of fermentor time required (effectively increasing the yield of PT). One of the limitations of this approach is that even when these species are supplied with an intact *ptx* operon, they do not consistently or efficiently export PT into the culture supernatant. This problem is overcome by using the *pts* genes of *B. pertussis* to enhance expression and export of PT in recombinant *B. parapertussis* or *B. bronchiseptica* containing the *ptx* operon. Complementation of *pts* functions in these species can be obtained by supplying the *pts* operon (or promoter fusions to *pts*) on an integrating plasmid. In the alternative, putative *pts* homologs found in the cells as obtained can be deleted and replaced with the *B. pertussis* genes. Many of the same manipulations that enhance expression of PT in *B. pertussis* (e.g., promoter fusions, increased *ptx* gene dosage, etc.) will function to enhance expression of PT in other recombinant *Bordetella* species when used in conjunction with *pts* complementation.

Recombinant strains of *B. pertussis* containing the *E. coli tac* promoter fused to the *ptx* operon export wild type levels of PT even in the presence of a modulating agent (MgSO₄) in the growth medium. Therefore, the presence of the tac promoter is sufficient to bypass any *Bvg*A requirement for export of PT. This is an encouraging finding with respect to the feasibility of expressing and exporting PT in heterologous systems.

One approach to obtaining expression and export of PT in heterologous systems which lack the Type II secretory pathway (Salmond, G.P.C. and P.J. Reeves, TIBS 18:7-12 (1993)) (used for export of PT by *B. pertussis*), is to supply the heterologous host (e.g., *E. coli*) with the export factors encoded by the *pts* operon (in addition to the *ptx* operon) on one or more plasmids. The feasibility of this approach is supported by previous work showing that Type II secretory pathways have been functionally reconstituted in *E. coli* (Possot, O. et al., Mol. Microbiol. 5:95-101 (1992); Cussac, V. et al., Microb. Ecol. Health Dis. 4:5139 (1991)). *P*_{*tac*}*-ptx ptsA* fusions may be sufficient for expression and export of PT in a heterologous system lacking the Type II secretory pathway, or additional promoter fusions to other *pts* encoded factors may be required. In the event that other *pts* encoded factors are required, clones containing the *P*_{*tac*}*-ptx* fusions contiguous with the entire *pts* operon are constructed. Although it is unlikely, in the event that reconstitution of export requires *Bvg* dependent functions, the host can be co-transformed with *bvgAS* containing plasmids constructed for this purpose.

Cloning the *ptx* and *pts* operons (containing promoter fusions to bypass *BvgA* function) on broad host range plasmids (Ditta, G. et al., Proc. Natl. Acad. Sci. USA 77:7347-7351 (1980) allows for screening a large number of alternate hosts for the ability to export PT. The advantage in utilizing other bacterial species for export of PT is that many of them may contain similar "Type II-like" secretory factors that may more efficiently complement the *pts* encoded factors of *B. pertussis*.

### Biological Deposits

PBCC558, PBCC562, DH5α,pPX2871 and DH5α,pPX2557 were deposited under the terms of the Budapest Treaty with the American Type Culture Collection (ATCC), 12301 Parklawn Drive, Rockville, Maryland 20852 on March 12, 1993 and have been assigned ATCC Accession Numbers 55402 , 55401 , 69255 and 69256, respectively. All restrictions upon the availability to the public of the deposited material will be irrevocably removed upon granting of a patent on this application. These deposits will be maintained in a public depository for a period of at least 30 years from the date of deposit or for the enforceable life of the patent or for the period of five years after the date of the most recent request for the furnishing of a sample of the biological material, whichever is longer. The deposits will be replaced if they should become nonviable or nonreplicable.

Those skilled in the art will recognize, or be able to ascertain using no more than routine experimentation, many equivalents to the specific embodiments described herein. Such equivalents are intended to be encompassed by the following claims.

**TABLE 1**

| Description of Bacterial Strains and Plasmids | |
|---|---|
| *B. pertussis* Strains^{a} | Description |
| PBCC524 | *ptx*Δ::Kan^{r} (Kan^{r} marker replacing *ptx* 5' region) |
| PBCC526 | spontaneous Str^{r} isolate of Tohama wild type |
| PBCC537 | *ptx*Δ4::Kan^{r}, (Kan^{r} marker replacing entire *ptx*) |
| PBCC554 | *ptxS*1-9K, 261^{b},Str^{r},*fha*B::Tet^{r} (*fha*B⁻ derivative of PBCC526 containing a double mutation in S1) |
| PBCC556 | Str^{r}, *fha*B::Tet^{r},Φ (P_{tac}-*ptx*)(*E. coli tac* promoter fused to *ptx* coding region) |
| PBCC558 | PBCC554 containing Δ*pts*A::Kan^{r} |
| PBCC561 | PBCC558 with integrated Amp^{r} pPX2833 |
| PBCC562 | PBCC554 containing *pts*B::Kan^{r} |
| PBCC563 | PBCC558 with Δ*pts*A::Kan^{r} replaced with wild type sequences |

| Plasmids^{c} | |
|---|---|
| pPX2557 | genomic clone in pUC18 containing 8kb 3' of *ptx* |
| pPX2558 | 4.5kb BamHi-Sall subclone from pPX2557 containing *pts*B in pUC18 |
| pPX2561 | Kan^{r} replacing 4.6kb EcoRI-BamHI region of *ptx* and *pts*A |
| pPX2727 | *ptx* operon contained on pRK290 derivative |
| pPX2777 | pNO1523 (Str^{s}) derivative |
| pPX2833 | 3.7kb BglII subclone from pPX2557 containing *pts*A in pUC18 |
| pPX2834 | Δ*pts*A::Kan^{r} (Kan^{r} insert in *pts*A of pPX2833) |
| pPX2856 | *pts*B::Kan^{r} (Kan^{r} insert in *pts*B of pPX2558) |
| pPX2857 | 3.76kb BglII subclone from pPX2557 containing *pts*A in pPX2777 |

| | |
|---|---|
| ^{a} All *B. pertussis* strains listed are hemolytic (Hly+) | |
| ^{b} PBCC554 and derived strains contain a double mutation (*ptx*S1-9K,261) in the *ptx*S1 gene encoding for Arg9 to Lys and Trp26 to IIe. | |
| ^{c} All plasmids except pPX2727 and pPX2777 are pUC based vectors. | |

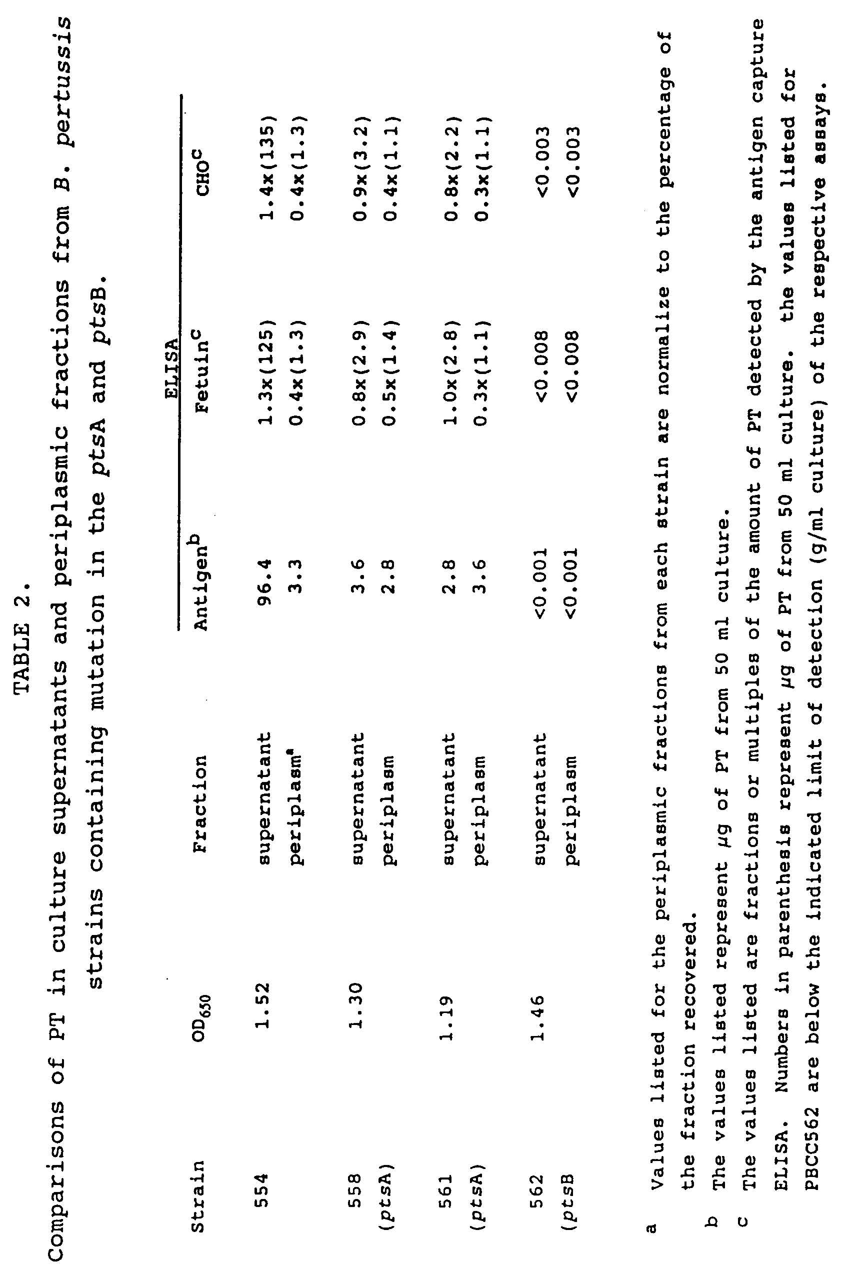

## Claims

1. Isolated DNA consisting essentially of the nucleotide sequence as shown in Fig. 1, SEQ ID NO: 1.

2. A plasmid construct containing the DNA of Claim 1.

3. Isolated DNA which hybridizes to all or a portion of the DNA sequence represented in SEQ ID NO: 1.

4. A plasmid construct comprising approximately 8kb of *Bordetella pertussis* nucleic acid sequences located 3' of *ptx* is the *Bordetella pertussis* genome, encoding gene products involved in expression or secretion of *Bordetella pertussis* holotoxin.

5. A plasmid construct of Claim 4, designated pPX2557, ATCC Deposit No. 69256.

6. Isolated DNA consisting essentially of the *Bordetella pertussis* sequences cloned in plasmid pPX2557, ATCC Deposit No. 69256.

7. A plasmid construct comprising an approximately 12.5kb region of *Bordetella pertussis* genome located approximately 5kb to approximately 17.5kb 3' of the beginning of *ptx* in the *Bordetella pertussis* genome.

8. A plasmid construct of Claim 7, designated pPX2871, ATCC Deposit No. 69255.

9. Isolated DNA consisting essentially of the *Bordetella pertussis* sequences cloned in plasmid pPX2871, ATCC Deposit No. 69255.

10. A host bacterium harboring the plasmid of Claim 4.

11. A host bacterium harboring the plasmid of Claim 7.

12. A host bacterium of Claim 10, representing a bacterial strain selected from the group consisting of: *Bordetella pertussis*, *Bordetella parapertussis*, *Bordetella bronchiseptica*, *Eschericia coli* and *Bacillus subtillis*.

13. A host bacterium of Claim 11, representing a bacterial strain selected from the group consisting of: *Bordetella pertussis*, *Bordetella parapertussis*, *Bordetella bronchiseptica*, E*schericia coli* and *Bacillus subtillis*.

14. A purified DNA sequence encoding all or a portion of the *Bordetella pertussis* gene, *pts*A.

15. A purified DNA sequence encoding all or a portion of the *Bordetella pertussis* gene, *pts*B.

16. A purified DNA sequence represented by SEQ ID NO: 1, encoding all or a portion of *Bordetella pertussis* genes *pts*A and *pts*B, including DNA sequences 3' of the reading frame encoding *pts*B, said purified DNA sequence useful for expression of pertussis holotoxin in *Bordetella pertussis*.

17. A gene product encoded by a DNA sequence represented by SEQ ID NO: 1, expressed from an open reading frame which starts approximately 697 base pairs 3' to the *ptx*S3 termination codon and encodes a predicted protein of 61kd.

18. A gene product encoded by an open reading frame located immediately 3' of the open reading frame encoding *pts*A, which open reading frame is designated *pts*B, and encodes a predicted protein of 32kd.

19. A method for regulating expression and secretion of pertussis holotoxin in a *Bordetella* species host, said method comprising the steps of:
a) providing the host with a plasmid construct comprising the *pts* operon operatively linked to an active promoter, and
b) maintaining the host under conditions appropriate for expression of *pts* from the active promoter.

20. A method of Claim 19, wherein the host is selected from the group consisting of *Bordetella pertussis*, *Bordetella parapertussis* and *Bordetella bronchiseptica*.

21. A method for regulating expression and secretion of pertussis holotoxin in a heterologous bacterial host, said method comprising the steps of:
a) providing the heterologous host with a plasmid construct comprising *ptx* and *pts* operatively linked to an active promoter; and
b) maintaining the heterologous host under conditions appropriate for expression and secretion of *ptx*.

22. A method of Claim 21 wherein the bacterial host is selected from the group consisting of *Eschericia coli* and *Bacillus subtillis*.


SEQUENCE LISTING

(1) GENERAL INFORMATION:

(i) APPLICANT: American Cyanamid Company

(ii) TITLE OF INVENTION: Expression of Pertussis Holotoxin in
Bordetella Pertussis

(iii) NUMBER OF SEQUENCES: 1

(iv) CORRESPONDENCE ADDRESS:
(A) ADDRESSEE: Hamilton, Brook, Smith & Reynolds, P.C.
(B) STREET: Two Militia Drive
(C) CITY: Lexington
(D) STATE: MA
(E) COUNTRY: U.S.A.
(F) ZIP: 01730

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US
(B) FILING DATE: 15-MAR-1993
(C) CLASSIFICATION:

(viii) ATTORNEY/AGENT INFORMATION:
(A) NAME: Carroll, Alice O
(B) REGISTRATION NUMBER: 33,542
(C) REFERENCE/DOCKET NUMBER: ACC93-01

(ix) TELECOMMUNICATION INFORMATION:
(A) TELEPHONE: (617) 861-6240
(B) TELEFAX: (617) 861-9540

(2) INFORMATION FOR SEQ ID NO:1:

(i) SEQUENCE CHARACTERISTICS:
(A) LENGTH: 2206 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO:1:
ATGAACCGGC GCGGCGGCCA GACCGCATTT GCGGCCATTG CGCGCAACGA GCGCGCCATC 60
GCTGCGTTCA TCCCCTACAG CAGCCACCTG ACGGACACGA CGCTGATCAC CCATGGCGCG 120
GACCTGGTCC GCACCTGGCG CGTACAGGGG ATCGCCTTCG AAAGCGCCGA GCCAGAGCTG 180
GTTTCGCAGC GCCATGAACA GCTCAACGGC CTGTGGCGCG CCATCTCGTG CGAGCAGGTC 240
GCGCTTTGGA TCCATTGCAT CCGGCGCAAG ACGCAGGCCG GGTTGGATGC GCGGTACGAA 300
AATCCGTTCT GCCGCGCGCT CGACGCCTCG TACAACGCCC GACTGAACGC GCGGCAGGCA 360
ATGACGAACG AATTCTACCT CACCCTGGTA TATCGGCCTG GCCACACCGC GCTCGGCAAG 420
CGTGCGCATC ACGGCCAGGC CGAGGTCCGC CGGCAACTGC TGGCCCATGT TCGACGCATG 480
GACGAAATCG GATCCCTGAT CGAAACGACA CTGCACAGCC ATGGCGAGAA CCACGAGCAG 540
ACCATCACCG TGCTGGGCTG CGAGACGGAC AACACCGGCC GGCGATACTC CCGGACGCTG 600
ACCCTGCTCG AATTCCTGCT CACCGGCCAC TGGCAACCGG TACGTGTGCC GACCGGGCCG 660
GTGGACGCGT ATCTCGATTC GAGCCGGATC CTTGCCGGCG CCGAAATGAT GGAGTTGCGT 720
GCTCCGACCT GCCGCCGCTA CGCGCAGTTC ATCGATTTCA AGGAATACGG CACGCACACC 780
GAACCAGGGA TGCTGAATGC CTTGCTGTAC GAGGATTACG AATATGTGAT CACGCATTCG 840
TTCAGCGCGG TCGGCAAGCG ACAGGCGCTG GCCTACCTGC AGCGGCAGCG CGCCCAGCTG 900
GCCAACGTGC AGGACGCCGC GTACTCGCAG ATCGACGACC TCGCGCATGC CGAAGACGCC 960
CTGGTCAATG GCGATTTCGT GATCGGCGAG TATCACTTCT CGATGATGAT CCTCGGCGCC 1020
GACCCCCGGC AACTGCGGCG CGATGTCAGT TCGGCCATGA CGCGCATCCA GGAGCGCGGC 1080
TTTCTCGCCA CGCCGGTGAC GTTGGCCCTG GATGCCGCCT TCTATGCGCA ATTGCCTGCC 1140
AACTGGGCAT ACCGGTCGCG CAAGGCCATG TTGACCAGCA GAAACTTCGC CGGACTGTGC 1200
AGCTTTCATA ATTTCTACGG CGGCAAGCGC GATGGCAACC CCTGGGGCCC GGCCCTGAGC 1260
CTGCTGTCCA CGCCTTCCGG GCAACCGTTC TACTTCAATT TCCATCACTC CGGGCTCGAC 1320
GAGGATTGCC GCGGCCAGAT GATGCTGGGC AACACGCGCA TCATCGGCCA GTCCGGCAGC 1380
GGCAAGACCG TGCTGCTCAA TTTCCTGCTT TGGCAGCTGC AGAAATTCCG ATCCGCGGAT 1440
GCCGATGGCC TGACGACGAT TTTCTTCGAC AAGGACCGGG GCGCGGAAAT CTGCATCCGA 1500
GCCCTCGATG GCCAGTACTG GCGGATACGC GACGGCGAAC CGACCGGTTC CAACCCCTGG 1560

AAGCTGCCAT GAACCGACCG AAATGTCATG TCCCTGGACT CGCTTCTGGC GATGCTCGCG 1620
CGCGCTCATG ACTCGCCGCT GACGTCGGCG CAGCACGCGA CGCTGGCCAC CGCTGTGCGC 1680
ACGGTGCTGC GCATGCCGGC GTCGCTGCGG CGAATGTCCA CGCTGCTGCA AAACATCACC 1740
CAGGCCACGT CCGAGCAGCG GGAACTGGTC AGACGCCTGG GGCGCTGGTG CCGCGACGAC 1800
GGCGCCGGTG GCACGGGAAT GCTGTGGTGG GTCTTCGACA ATCCGAATGA TTGCCTCGAT 1860
TTTTCGCGGC CGGGCAACTA CGGCATCGAC GGCACCGCGT TCCTGGACAA TGCCGAGACG 1920
CGCACGCCGA TCTCGATGTA CCTGTTGCAT CGGATGAACG AGGCCATGGA TGGACGGCGC 1980
TTCGTCTATC TCATGGACGA AGCCTGGAAG TGGATCGACG ACCCGGCCTC CGCCGAGTCC 2040
GCAGGCGACC AGCAGCTGAC CATACGCAAG AAGAACGGGC TGGGCGTCTC CTCCACGCAA 2100
ATGCCAAGCA GCCTGCTCGG CGCGAGGGTC GCCGCATCGC TGGTACAGCA ATGCGCAACC 2160
GAGATCTATC TGCCCAGGCC GACGGCCAAT CCGATGGTTC AAATAA 2206